# EUROPEAN PATENT APPLICATION

(11) **EP 3 677 672 A1**
(43) Date of publication of application: **08.07.2020**
(21) Application number: 18850438.5
(22) Date of filing: 23.08.2018
(51) Int. Cl.: C12N 7/01

(54) **METHOD FOR GRADUAL CONSTRUCTION OF REASSORTANT INFLUENZA VIRUS**

(30) Priority: 28.08.2017 JP 2017163114
(71) Applicant: The Research Foundation for Microbial Diseases of Osaka University, Suita-shi, Osaka 565-0871 (JP)
(72) Inventor: FUJIMOTO, Takao, Kanonji-shi Kagawa 768-0065 (JP)
(74) Representative: Hertin und Partner Rechts- und Patentanwälte PartG mbB
(86) International application number: PCT/JP2018/031072
(87) International publication number: WO 2019/044636

(57) **Abstract**

Provided is a production method for reassortant influenza virus having genome segments of two or more kinds of influenza virus in the case where an antigenic strain and donor strain have similar antigenicities. The production method makes use of the first influenza virus (1) containing an antigenic protein (x), the second influenza virus (2) having an antigenic protein (x') having antigenicity similar to that of the strain (1), and the third influenza virus (3) having an antigenic protein (y) having antigenicity different from that of the strain (1), and includes the steps of: coculturing the strain (2) and the strain (3) by infecting a host therewith, to produce reassortant influenza viruses; selecting influenza virus (Y) having the antigenic protein (y) from the viruses; then coculturing the strain (1) and the selected strain (Y) by infecting a host therewith; and selecting influenza virus (X) having the antigenic protein (x) from reassortant influenza viruses produced from the strain (1) and the strain (Y).

## Description

### Technical Field

The present invention relates to a production method for reassortant influenza virus having genome segments of two or more kinds of influenza virus, and more specifically, to reassortant influenza virus production method including at least two reassortment steps.

The present application claims priority from Japanese Patent Application No. 2017-163114, which is incorporated herein by reference.

### Background Art

Influenza is an infectious disease causing epidemics all over the world every year, and is caused by influenza virus. Influenza virus belongs to the family Orthomyxoviridae, and has an envelope having lipid bilayer structure. Influenza viruses are classified into three groups, i.e., type A, type B, and type C, which are referred to as influenza A virus, influenza B virus, and influenza C virus, respectively. Influenza virus generally refers particularly to type A or type B. Differences between type A, type B, and type C are based on differences in antigenicity of M1 protein and NP protein among proteins constituting virus particles. In addition, even influenza viruses of the same type A or type B are classified into pluralities of subtypes and strains on the basis of differences in antigenicity of hemagglutinin (hereinafter referred to as "HA") and neuraminidase (hereinafter referred to as "NA"), which are molecules on the surface of the envelope.

Influenza virus undergoes an antigenic change with high probability to generate a new type of influenza virus. Influenza A Virus is classified into 16 HA (H1 to H16) subtypes and 9 NA (N1 to N9) subtypes on the basis of the antigenicity of HA and NA thereof. Three HA (H1, H2, and H3) subtypes of influenza A virus are particularly important pathogens. H1N1 subtype and H3N2 subtype of influenza A virus spread seasonally and cause human infection. In 2003, influenza virus classified into H5 subtype, which was highly lethal and of avian origin, emerged as a human pathogen. H1N1 subtype virus emerged as a new type of influenza virus in April 2009, and has spread rapidly among human population. Influenza may even cause a pandemic, and hence there is a demand that influenza vaccine be quantitatively secured.

For manufacture of the influenza vaccine, methods involving growing influenza virus through utilization of embryonated chicken eggs are used. In addition, methods involving growing the influenza virus in cultured cells are beginning to be put into practical use as well. Influenza vaccine strain is selected by predicting epidemics in next year's season on the basis of, for example, an epidemic situation in Japan obtained under the National Epidemiological Surveillance of Infectious Diseases Program and information on viruses isolated in Japan, such as antigenicity and results of genetic analysis. However, some strains may have low infectious titers in culture supernatants, and improvement in growth potential is an important issue. When the embryonated chicken eggs or the cultured cells are utilized for growing such influenza virus, there is a problem in that growth potential of the virus in the host is reduced depending on the subtype or strain of the influenza virus. Therefore, attempts have been made to produce a recombinant of influenza virus having improved growth potential in the host by recombination technology. Examples of the recombination technology include reassortment method and reverse genetics method (hereinafter referred to as "RG method"). One example of RG method is a method of producing a recombinant of influenza virus, involving simultaneously introducing a total of 12 kinds of plasmids, specifically 8 kinds of plasmids (Poll plasmids) for supplying viral RNAs (vRNAs), and 4 kinds of expression plasmids (PolII plasmids) encoding structural proteins needed for forming virus particles, into cells (Non Patent Literature 1).

For cell-culture influenza vaccine, seed viruses showing high growth potential in cultured cells are desirably used, and efficient production of the seed virus is needed for stable supply of the vaccine. In Patent Literature 1, there is a disclosure that reassortant virus produced by RG method with the use of nucleotides having backbone sequences from the same influenza virus A subtype as that of antigenic strain and nucleotides having an attenuating mutation introduced into sequence of HA showed high growth potential in cells. However, RG method places heavy burden on the host cells owing to the simultaneous introduction of several plasmids into the cells. In addition, RG method has a problem in that it takes time to prepare various plasmids, and hence it is difficult to quickly produce a recombinant.

The reassortment method, in which the host is coinfected with at least two or more kinds of influenza virus, and their genome segments are exchanged and reassorted in growth process, to thereby produce a recombinant, is under investigation as a recombination technology for influenza virus. An attempt has been made to produce, by the reassortment method, reassortant influenza virus containing genome segments encoding proteins having desired antigenicities and genome segments encoding desired backbone proteins.

In the reassortment method, the host is coinfected with two or more kinds of influenza virus, and their genome segments are exchanged and reassorted in growth process, to thereby produce a recombinant (Non Patent Literatures 2 and 3). The production of recombinant influenza virus by reassortment method has been performed using chicken eggs as the host. Specifically, an embryonated chicken egg is subjected to mixed infection with donor strain having high growth potential, such as PR8 strain, and epidemic strain (antigenic strain), to thereby produce a recombinant having both backbone genes of high growth potential and antigen genes of epidemic strain. However, the reassortment method in which the embryonated chicken egg is utilized as the host has a problem in that recombinant of interest cannot always be produced. For the purpose of obtaining a recombinant of influenza virus, reassortment method using cultured cells is under consideration. Also in the reassortment method using cultured cells, there is a concern that a recombinant of interest cannot always be produced. In Patent Literature 2, as the reassortment method using cultured cells, there is a disclosure that a host infected with two kinds of influenza virus is brought into contact with an inhibitory agent capable of inhibiting transcription or translation of HA and/or NA of donor strain, to thereby produce reassortant influenza virus.

### Citation List

### Patent Literature

[PTL 1] JP 5686741 B2
[PTL 2] WO 2011/145081 A1

Non Patent Literature

[NPL 1] Neumann et al., PNAS Vol. 102, p. 16825-16829 (1999)
[NPL 2] PLoS Pathog. 2015 Oct; 11(10): e1005204.
[NPL 3] J Virol. 1976 Oct; 20(1): 248-54.

### Summary of Invention

### Technical Problem

An object of the present invention is to provide a production method for reassortant influenza virus having genome segments of two or more kinds of influenza virus in the case where antigenic strain and donor strain have similar antigenicities, and influenza virus obtained thereby.

### Solution to Problem

The inventor of the present invention has made extensive investigations in order to achieve the above-mentioned object, and as a result, has found that the object can be achieved by a method including at least two reassortment steps making use of at least three kinds of influenza virus. Thus, the inventor has completed the present invention.

That is, the present invention includes the following.
1. A production method for influenza virus (X) containing an antigenic protein (x), which is a production method for reassortant influenza virus, the production method comprising at least two reassortment steps including the following Step (A) and Step (B) making use of at least three kinds of influenza virus including following (1) to (3):
   (1) the first influenza virus containing an antigenic protein (x);
   (2) the second influenza virus having an antigenic protein (x') having similar antigenicity to that of influenza virus of (1); and
   (3) the third influenza virus having an antigenic protein (y) having antigenicity different from that of influenza virus of (1) :
      Step (A): the step including:
         coculturing influenza virus (2) and influenza virus (3) by infecting a host therewith, to produce reassortant influenza viruses; and
         selecting influenza virus (Y) having the antigenic protein (y) from reassortant influenza viruses; and
      Step (B): the step including:
         coculturing influenza virus (1) and influenza virus (Y) produced in Step (A) by infecting a host therewith, to produce reassortant influenza viruses; and
         selecting influenza virus (X) having the antigenic protein (x) from reassortant influenza viruses.
2. The production method for influenza virus (X) according to the above-mentioned item 1, further comprising, before the coculturing influenza virus (2) and influenza virus (3) in Step (A), the step of treating influenza virus (3) so that influenza virus has initial infection ability and loses or is reduced in growth potential.
3. The production method for influenza virus (X) according to the above-mentioned item 1 or 2, further comprising, before the coculturing influenza virus (1) and influenza virus (Y) in Step (B), the step of treating influenza virus (1) so that influenza virus has initial infection ability and loses or is reduced in growth potential.
4. The production method for influenza virus (X) according to any one of the above-mentioned items 1 to 3, wherein the step of selecting influenza virus (Y) having the antigenic protein (y) in Step (A) comprises the step of bringing an antibody reactive to the antigenic protein (x') into contact therewith.
5. The production method for influenza virus (X) according to any one of the above-mentioned items 1 to 4, wherein the step of selecting influenza virus (X) having the antigenic protein (x) in Step (B) comprises the step of bringing an antibody reactive to the antigenic protein (y) into contact therewith.
6. The production method for influenza virus (X) according to any one of the above-mentioned items 1 to 5, wherein Step (A) includes selecting influenza virus (Y) having the antigenic protein (y) from reassortant influenza viruses.
7. The production method for influenza virus (X) according to any one of the above-mentioned items 1 to 6, wherein Step (B) includes selecting influenza virus (X) having the antigenic protein (x) from reassortant influenza viruses.
8. Influenza virus (X), which is produced by the production method of any one of the above-mentioned items 1 to 7.
9. Reassortant influenza virus, including proteins derived from at least two kinds of influenza virus including antigenic strain influenza virus and donor strain influenza virus, wherein antigenic strain influenza virus and donor strain influenza virus have similar antigenicities.

### Advantageous Effects of Invention

According to the production method for reassortant influenza virus of the present invention, reassortant influenza virus having genome segments of two or more kinds of influenza virus can be produced in the case where antigenic strain and donor strain have similar antigenicities.

### Brief Description of Drawings

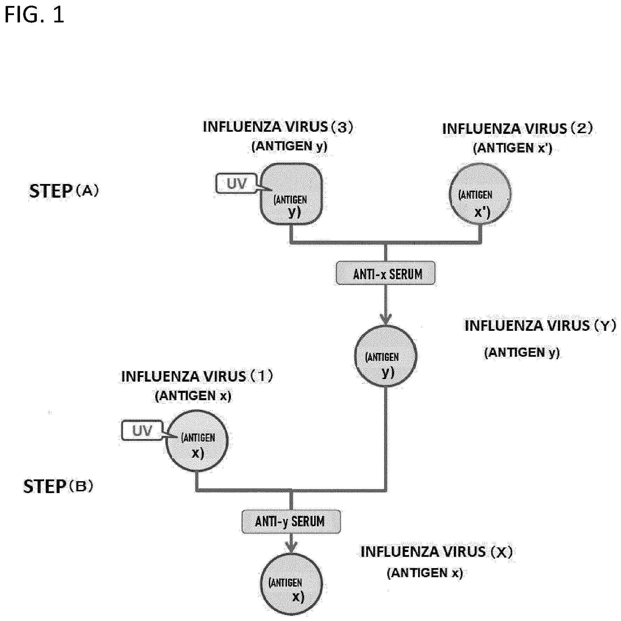

FIG. 1 is a diagram for illustrating the concept of the production method for reassortant influenza virus of the present invention.

### Description of Embodiments

The present invention relates to a method of producing reassortant influenza virus having genome segments of two or more kinds of influenza virus in the case where antigenic strain and donor strain have similar antigenicities.

Influenza virus has an envelope having lipid bilayer structure. The inner layer of the envelope is mainly formed of matrix protein and RNP, which is a complex of RNA and proteins. Influenza virus has eight genes (genome segments), specifically PB2, PB1, PA, HA, NP, NA, M, and NS, and the outer layer is covered with NA and HA, which are major antigenic proteins. The HA and NA genome segments encode HA and NA antigenic proteins, respectively, and the other six genome segments, which are the PB2, PB1, PA, NP, M, and NS segments, encode backbone proteins.

As used herein, the term "protein having antigenicity" (hereinafter "antigenic protein") refers to a protein expressed from any one of the genome segments encoding HA and NA, and the term "backbone protein" refers to a protein expressed from any one of the following six genome segments: PB2, PB1, PA, NP, M, and NS segments. Herein, influenza virus having a genome segment encoding a desired antigenic protein is referred to as "antigenic strain". In addition, herein, influenza virus having a genome segment encoding desired backbone proteins is referred to as "donor strain".

Reassortment method has a significant problem in that its recombination efficiency is low, resulting in a high probability that a recombinant of interest cannot be obtained. Meanwhile, in Patent Literature 2 mentioned in the "Background Art" section, there is a description that it takes about 35 days to obtain reassortant influenza virus having high growth potential. That is, although the reassortment method can reduce the time and cost required for the preparation of various plasmids and cells as compared to an RG method by virtue of producing a recombinant using virus itself, there is a concern that it may take a long period of time to obtain reassortant influenza virus having high growth potential of interest as a result of the low recombination efficiency.

The inventor of the present invention has considered that the failure to obtain target recombination efficiency in the reassortment method is caused by an inability to control genome segment exchange occurring during the growth process in coinfected influenza viruses. Therefore, in the production of influenza virus having desired antigenicity and high growth potential by the reassortment method using the first influenza virus having desired antigenicity and the second influenza virus having a high-growth-potential backbone, the inventor made an investigation on early and efficient production of reassortant influenza virus by coculturing the first influenza virus treated so as to have initial infection ability and lose or be reduced in growth potential and the second influenza virus, and selecting virus having the antigenicity of the first influenza virus. Here, an example of the treatment for allowing the first influenza virus to have initial infection ability and lose or be reduced in growth potential is ultraviolet light irradiation. Ultraviolet light irradiation dose only needs to be such an irradiation dose as to allow influenza virus to have initial infection ability and allow influenza virus to lose or be reduced in growth potential.

However, as described above, influenza is an infectious disease causing epidemics all over the world every year, and undergoes change in antigenicity at a high probability. Accordingly, it is difficult to predict the influenza virus strain that is to cause an epidemic in a present year (hereinafter referred to as "epidemic strain"). In attempting recombination in order to enhance the growth potential of epidemic strain in a desired host, the inventor of the present invention has found that, when donor strain having backbone proteins excellent in growth potential and epidemic strain have similar antigenicities, it is difficult to produce a recombinant by the related-art reassortment method. Therefore, there is a need for a method by which reassortant influenza virus can be produced even in the case where the antigenic strain and donor strain have similar antigenicities. In view of the foregoing, the inventor has found that reassortant influenza virus can be produced by a method including at least two reassortment steps making use of at least three kinds of influenza virus. Thus, the inventor has completed the present invention. That is, the present invention relates to a production method for influenza virus (X) containing an antigenic protein (x), which is a production method for influenza virus using a production method for reassortant influenza virus, the production method including at least two reassortment steps including the following Step (A) and Step (B) making use of at least three kinds of influenza virus including the following (1) to (3) (see FIG. 1).

At least three kinds of influenza virus including the (1) to (3) to be used herein, influenza virus (X) of the present invention, and influenza virus (Y) to be produced before the production of influenza virus (X) are described. Influenza virus of interest of the present invention is influenza virus containing a desired antigenic protein (x) and having desired backbone proteins.

Herein, influenza virus (1) is the first influenza virus (antigenic strain) having the desired antigenic protein (x). Herein, influenza virus (2) is the second influenza virus (donor strain) having: an antigenic protein (x') having antigenicity similar to that of the antigenic protein (x); and the desired backbone proteins. Herein, influenza virus (3) is the third virus having an antigenic protein (y) having antigenicity different from that of the antigenic protein (x).

Herein, influenza virus (Y) refers to reassortant influenza virus that is produced by coculturing influenza virus (2) and influenza virus (3) by infecting a host therewith, and that is reassortant donor strain having the antigenic protein (y) and the desired backbone proteins.

Herein, influenza virus (X) refers to reassortant influenza virus that is produced by coculturing influenza virus (1) and influenza virus (Y) by infecting a host therewith, and that is influenza virus of interest of the present invention having the desired antigenic protein (x) and the desired backbone proteins.

Influenza virus (X) that is influenza virus of interest to be obtained in the present invention is one in which at least one of genome segments encoding HA and NA (preferably at least the genome segment encoding HA) is derived from influenza virus (1) and at least one of the other genome segments is derived from influenza virus (2).

The production method for influenza virus (X) containing an antigenic protein (x) of the present invention includes at least two reassortment steps including following Step (A) and Step (B).

Step (A): The step including: coculturing influenza virus (2) and influenza virus (3) by infecting a host therewith, to produce reassortant influenza viruses; and selecting influenza virus (Y) having the antigenic protein (y) from reassortant influenza viruses.

### Step (A)-1: The step of Inactivating Influenza Virus (3)

Prior to the production of reassortant influenza viruses, influenza virus (3) is treated so as to have initial infection ability, and to lose or be reduced in growth potential. Specifically, influenza virus (3) is irradiated with ultraviolet light to inactivate influenza virus. The irradiation dose of the ultraviolet light is preferably such that influenza virus after the ultraviolet light irradiation has initial infection ability for the host, but its growth potential after infection is lost or reduced. That the growth potential after infection is lost or reduced means that, when the host is infected with the first influenza virus alone, the growth potential of the virus in the host is not confirmed, or the growth potential is reduced as compared to that of the intact first influenza virus that has not been subjected to the ultraviolet light irradiation. The growth potential may be evaluated by using a known index, such as virus infectious titer or Plaque Forming Unit (PFU). In addition, when the host is infected with the first influenza virus after the ultraviolet light irradiation, the first influenza virus needs to have infectivity for the host, namely the initial infection ability. When the host is cultured cells, a state of having the initial infection ability means that cytopathic effect (CPE) caused by the virus subjected to the ultraviolet light irradiation is observed. In this step, it is preferred that the first influenza virus be irradiated with ultraviolet light irradiation dose equivalent to that in the case where ultraviolet light irradiation is performed in the Time Mode of Spectrolinker XL-1000 (Spectronics Corporation) for from 1 to 60 seconds, preferably from 5 to 50 seconds, still more preferably from 10 to 40 seconds, most preferably from 10 to 30 seconds. The irradiation conditions, such as the apparatus to be used for such UV light irradiation (UV light intensity, distance from a light source, and the like are described in Examples below) and the irradiation time, are mere examples, and those conditions may be appropriately adjusted/changed as long as ultraviolet light irradiation dose comparable to that under the irradiation conditions is achieved. The ultraviolet light irradiation dose under the above-mentioned conditions enables influenza virus having initial infection ability for the host but having its growth potential lost or reduced to be efficiently obtained, and hence is preferred. In the present invention, by virtue of causing the growth potential of the first influenza virus to be lost or reduced while having initial infection ability for the host, recombination efficiency in the host can be improved.

### Step (A)-2: The step of Producing Reassortant Influenza Viruses

Reassortant influenza viruses of influenza virus (2) and influenza virus (3) may be produced by coculturing influenza virus (2) and influenza virus (3) subjected to the ultraviolet light irradiation by infecting a host therewith.

The host may be infected with influenza viruses simultaneously or not simultaneously. It is preferred that the host be infected with influenza virus (3), and then infected with influenza virus (2). The infection of the host with each of influenza viruses is performed by bringing the host and influenza virus into contact with each other. Influenza virus (3) is preferably brought into contact with the host at preferably moi of from 1×10⁻⁶ to 10, more preferably moi of from 0.001 to 1, still more preferably moi of from 0.1 to 1. Influenza virus (2) is preferably brought into contact with the host at preferably moi of from 0.001 to 10, more preferably moi of from 0.01 to 1, still more preferably moi of from 0.1 to 1. Hitherto, in order to coinfect a host with influenza viruses, it has been required that the host be infected by bringing the viruses into contact therewith at high concentrations. However, in the present invention, even at low concentrations, influenza viruses coinfect the host to allow a recombinant to be efficiently produced. The moi of influenza virus (3) is a value before the irradiation with ultraviolet light. The infectious titer (TCID₅₀/mL) of each of influenza viruses may be confirmed in accordance with the method disclosed in "Part IV" of "Influenza Diagnosis Manual (3rd edition, September 2014)" written by the National Institute of Infectious Diseases, Japan (hereinafter referred to as "Reference 1"), and the moi may be calculated by dividing the infectious titer by the number of cells.

The host infected with influenza virus (3) and influenza virus (2) is cultured to provide a culture product. Through the culture of this step, influenza viruses are reassorted in the host. Culture conditions for the host, such as culture temperature, may be any conditions as long as the conditions allow influenza viruses to grow in the host. When the host is cultured cells, the medium to be used for the culture is preferably liquid medium. Serum of animal origin is often added to liquid medium, but the possibility cannot be denied that the serum of animal origin contains an agent that inhibits the growth of influenza virus of interest. Therefore, serum-free medium that does not contain the agent is more preferably used. Examples of the serum-free medium include Eagle's MEM medium (Nissui Pharmaceutical), OptiPRO SFM (Thermo Fisher Scientific), VP-SFM (Thermo Fisher Scientific), EX-CELL MDCK (SAFC Biosciences), UltraMDCK (Lonza), ProVero 1 (Lonza), and BalanCD MDCK (Irvine Scientific). Culture time is preferably from 1 to 5 days, more preferably from 2 to 3 days. In this step, the culture product is obtained after the culture. The culture product contains reassortant influenza viruses reassorted in the host. Reassortant influenza viruses are contained in the allantoic fluid in the case where the host is an embryonated chicken egg, and are contained in the culture supernatant in the case where the host is cultured cells.

### Step (A)-3: The step of Selecting Influenza Virus (Y)

The selection of influenza virus (Y) having the antigenic protein (y) from reassortant influenza viruses produced in Step (A)-2 is achieved by inactivating influenza virus containing the antigenic protein (x') among reassortant influenza viruses in a culture product. The inactivation of influenza virus containing the antigenic protein (x') may be achieved using a physical technique, a chemical technique, or any other technique, but is preferably achieved by treating reassortant influenza viruses produced in Step (A)-2 with an antibody reactive to the antigenic protein (x'). The obtained culture product itself may be treated with the antibody.

The virus amount in the culture product to be subjected to this step may be represented by the product of virus infectious titer (TCID_{50/}mL) and dose (mL). As long as the culture product contains reassortant influenza virus of interest, the virus amount may be of any value, but is preferably 10² TCID₅₀ or more, more preferably 10³ TCID₅₀ or more, still more preferably 10⁴ TCID₅₀ or more. In addition, the virus amount may be appropriately adjusted through dilution or concentration by a known technique.

The antibody only needs to be reactive to the antigenic protein (x'), and may be polyclonal antibodies or monoclonal antibodies. Antiserum against influenza virus (2) may be used as the antibody. The antiserum is preferably added to the culture product at such a concentration as to give final dilution factor of preferably from 2 to 1,000 times, more preferably from 4 to 10 times. When the concentration falls within such range, the antiserum can suitably react to the antigenic protein of influenza virus (2) to efficiently inactivate influenza virus having the antigenic protein.

The antiserum against influenza virus (2) may be prepared by a known technique, and may be immune serum or infected serum. Infected serum is preferably selected. Such antiserum may be prepared by a known technique. The antiserum may be obtained by administering influenza virus (2) to a mammal or infecting the mammal with influenza virus (2), and then collecting blood from the mammal. For example, a mammal, such as a rabbit, a goat, a sheep, a mouse, or a rat, is immunized through the administration of influenza virus (2) as an immunogen. As administration means, intraperitoneal injection, intravenous injection, subcutaneous injection, or the like is adopted, and intradermal injection is also adopted in some cases. Booster immunization is repeated several times, blood is collected from the mammal 3 to 10 days after final immunization, and the immune serum may be obtained therefrom. In addition, for example, a mammal, such as a ferret or a mouse, may be infected with influenza virus (2). As an infection method, a method such as spray inoculation or nasal inoculation is adopted. Blood is collected from the mammal on or after the 10th to 14th day after the infection, and the infected serum may be obtained therefrom.

The obtained antiserum has preferably been inactivated its neutralizing activity nonspecific to the antigen derived from influenza virus (2) by a known technique, such as Receptor Destroying Enzyme (RDE) treatment, trypsin treatment, or potassium periodate treatment.

The antibody titer of the neutralizing antibody is preferably measured in advance. The antibody titer may be measured by a known technique, such as particle agglutination method (PA), indirect fluorescent antibody method (IFA), immune adherence hemagglutination method (IAHA), neutralization method (NT), hemagglutination inhibition method (HI), complement fixation method (CF), enzyme immunoassay (EIA), radioimmunoassay (RIA), chemiluminescence immunoassay (CLIA), or latex agglutination turbidimetry (LA). In an embodiment in which the virus infectious titer of the culture product is from 10⁷ to 10⁸ TCID₅₀/100 µL, antiserum showing an antibody titer measured by HI method of 10 or more, preferably 12.8 or more, more preferably 80 or more, still more preferably 128 or more may be used. When the antibody titer falls within such range, the antigenic protein of influenza virus (2) present in the culture product and the neutralizing antibody suitably bind to each other, and hence influenza virus having the antigenic protein can be efficiently inactivated.

Influenza virus (Y) of interest may be collected by treating the culture product containing reassortant influenza viruses produced in Step (A)-2 with the antibody reactive to the antigenic protein (x') and collecting reassortant influenza viruses in which influenza virus containing the antigenic protein (x') has been inactivated. Specifically, a mixture of the culture product and the neutralizing antibody is brought into contact with the host, and the infected host is cultured under suitable conditions described in Step (A)-2 to selectively grow reassortant virus of interest. When the host is cultured cells, cytopathic effect (CPE) caused by reassortant virus of interest is confirmed. Influenza virus (Y) can be more accurately selected by analyzing genome segments. A known technique may be used as an analysis method for the genome segments.

Step (B): The step including: coculturing influenza virus (1) and influenza virus (Y) produced in Step (A) by infecting a host therewith, to produce reassortant influenza viruses; and selecting influenza virus (X) having the antigenic protein (x) from reassortant influenza viruses.

### Step (B)-1: The step of Inactivating Influenza Virus (1)

In the same manner as in Step (A)-1, prior to the production of reassortant influenza viruses, influenza virus (1) is treated so as to have initial infection ability, and to lose or be reduced in growth potential. Influenza virus is irradiated with ultraviolet light to be inactivated. For treatment conditions under which the growth potential is lost or reduced, the conditions of Step (A)-1 may be referred to.

### Step (B)-2: The step of Producing Reassortant Influenza Viruses

Reassortant influenza viruses of influenza virus (1) and influenza virus (Y) may be produced by coculturing influenza virus (Y) and influenza virus (1) subjected to the ultraviolet light irradiation by infecting a host therewith. For coculture conditions, the conditions of Step (A)-2 may be referred to.

The infection of the host with each of influenza viruses is performed by bringing the host and influenza virus into contact with each other. Influenza virus (1) is preferably brought into contact with the host at preferably moi of from 1×10⁻⁶ to 10, more preferably moi of from 0.001 to 1, still more preferably moi of from 0.1 to 1. Influenza virus (Y) is preferably brought into contact with the host at preferably moi of from 0.001 to 10, more preferably moi of from 0.01 to 1, still more preferably moi of from 0.1 to 1. Hitherto, in order to coinfect a host with influenza viruses, it has been required that the host be infected by bringing influenza viruses at high concentrations into contact therewith. However, in the present invention, even at low concentrations, the influenza viruses coinfect the host to allow a recombinant to be efficiently produced.

The host infected with influenza virus (1) and influenza virus (Y) is cultured to provide a culture product. For culture conditions, the conditions of Step (A)-2 may be referred to.

### Step (B)-3: The step of Selecting Influenza Virus (X)

The selection of influenza virus (X) having the antigenic protein (x) from reassortant influenza viruses produced in Step (B)-2 is achieved by inactivating influenza virus containing the antigenic protein (y) among reassortant influenza viruses in a culture product. The inactivation of influenza virus containing the antigenic protein (y) is specifically achieved by treating reassortant influenza viruses produced in Step (B)-2 with an antibody reactive to the antigenic protein (y). The obtained culture product itself may be treated with the antibody. The antibody only needs to be reactive to the antigenic protein (y), and may be polyclonal antibodies or monoclonal antibodies. Antiserum against influenza virus (Y) may be used as the antibody. The antiserum is preferably added to the culture product at such a concentration as to give a final dilution factor of preferably from 2 to 1,000 times, more preferably from 4 to 10 times. When the concentration falls within such range, the antiserum can suitably react to the antigenic protein of influenza virus (Y) to efficiently inactivate reassortant influenza virus having the antigenic protein. The antiserum against influenza virus (Y) may be obtained by being prepared by the same technique as that for the antiserum against influenza virus (2).

Influenza virus (X) of interest may be collected by treating the culture product containing reassortant influenza viruses produced in Step (B)-2 with the antibody reactive to the antigenic protein (y) and collecting reassortant influenza viruses in which influenza virus containing the antigenic protein (y) has been inactivated. Specifically, a mixture of the culture product and the neutralizing antibody is brought into contact with the host, and the infected host is cultured under suitable conditions described in Step (A)-2 to selectively grow reassortant virus of interest. When the host is cultured cells, cytopathic effect (CPE) caused by reassortant virus of interest is confirmed. Influenza virus (X) can be more accurately selected by analyzing genome segments. A known technique may be used as an analysis method for the genome segments.

In the production method of the present invention, the inactivation of influenza virus refers to a state in which the growth potential of influenza virus is suppressed. The suppression of the growth potential refers to a state in which, when the infectious titer of virus to be measured by a general infectious titer measurement method typified by plaque method or TCID₅₀ method is reduced to the level below the detection limit and the virus is cultured with an appropriate substrate, the infectious titer reaches the level below the detection limit even within culture period of from 2 to 3 days. Means for suppressing the growth potential is not particularly limited, but the suppression may be achieved by treating the virus with an antibody reactive thereto.

Herein, the ease of production of a recombinant of influenza virus is indicated by recombination efficiency. The recombination efficiency refers to the ratio of the number of clones of a plaque that is influenza virus (X) to the total number of clones isolated as plaques in a reassortant virus production experiment. The reassortant virus production experiment means an experiment involving infecting a host with two kinds of influenza virus to produce reassortant influenza virus. According to the production method of the present invention, a recombination efficiency of preferably 60% or more, more preferably 80% or more, still more preferably 95% or more, most preferably 100% can be achieved.

Influenza virus (1), (2), or (3) of the present invention is not particularly limited, and may be selected as appropriate for reassortant influenza virus of interest. For example, influenza virus may be selected from all currently known subtypes, and subtypes to be isolated and identified in the future. In the case of influenza A virus, influenza viruses including combinations of various HA subtypes and NA subtypes are conceivable. In the case of influenza B virus, influenza viruses including the combination of Victoria lineage and Yamagata lineage are conceivable.

Each influenza A virus subtype has high RNA genome variability, and hence new strains are frequently generated. Influenza that is said to have caused a global outbreak after being recognized as causing an outbreak in Mexico in April 2009 is called novel influenza, swine influenza, pandemic influenza A (H1N1), swine flu, A/H1N1 pdm, or the like. Novel influenza, which is said to have spread among humans after its virus, which had caused an epidemic among swine, directly infected humans from swine at farms and the like, is distinguished from influenza A virus subtype H1N1 serving as Russian influenza A (hereinafter referred to as "H1N1 subtype") and influenza A virus subtype H3N2 serving as Hong Kong influenza A (hereinafter referred to as "H3N2 subtype"), which had existed earlier and were seasonal. In addition, because of the high RNA genome variability, even in the same influenza A virus subtype, virus strains are distinguished from each other on the basis of the time and place of isolation.

Influenza B virus continues to undergo irreversible antigenic drift, but mutates relatively slower than influenza A virus, and has epidemic cycle of about 2 years. Influenza B virus was isolated for the first time during an endemic in New York in 1940, and has often repeated epidemics since then, and a consequent increase in mortality rate has also been recorded. Influenza B virus has been observed to infect only humans, but has no subtypes, and has only two lineages, i.e., Yamagata lineage and Victoria lineage.

Herein, influenza virus (1), (2), or (3) may be a currently isolated and identified strain or a strain to be isolated and identified in the future, and may be type A or type B. For example, with regard to currently isolated and identified strains, influenza A virus is classified into 16 HA (H1 to H16) subtypes and 9 NA (N1 to N9) subtypes on the basis of the antigenicity of HA and NA thereof.

Influenza virus (1) only needs to be a strain containing the desired antigenic protein (x), and is not particularly limited. For example, a strain selected as a vaccine strain may be used. Specific examples thereof include type A strains (A/California/7/2009 (X-179A) (H1N1) pdm09 and A/Switzerland/9715293/2013 (NIB-88) (H3N2)) and type B strains (B/Phuket/3073/2013 (Yamagata lineage) and B/Texas/2/2013 (Victoria lineage)) that were selected as strains for the 2015/2016 season. In addition, any strain to be selected in the future may also be used.

It is preferred that each of influenza viruses (2) and (3) have an antigenic protein conforming to the conditions of the antigenic protein (y) or (x'), and have backbone proteins conforming to the purpose of the present invention. In particular, in order to use influenza virus (X) produced by the method of the present invention as a seed virus for influenza vaccine, it is desired that influenza virus (X) have growth potential in a desired host. In particular, it is preferred that influenza virus (2) has backbone proteins excellent in growth potential in a desired host. Specifically, when the host is chicken eggs, influenza virus is preferably of an H1N1 subtype. An example of the H1N1 subtype is A/Puerto Rico/8/34 (H1N1). Meanwhile, when the host is cultured cells, in particular, MDCK cells, influenza virus is preferably of an H3N2 subtype. Examples of the H3N2 subtype include A/Ibaraki/N12232/2012 (H3N2), A/Hiroshima/52/2005 (H3N2), and A/Panama/2007/99 (H3N2).

As influenza virus (1) or (3), a strain having an antigenic protein of interest only needs to be used without any particular limitation. Influenza virus may be a currently isolated and identified strain or a strain to be isolated and identified in the future, and may be influenza A virus or influenza B virus. Specific examples of influenza virus (1), (2), or (3) include, but not limited to, A/California/7/2009 (H1N1) pdm09, A/California/4/2009 (H1N1) pdm09, A/New Caledonia/20/99 (H1N1), A/Solomon Islands/3/2006 (H1N1), A/Brisbane/59/2007 (H1N1), A/Panama/2007/99 (H3N2), A/Wyoming/3/2003 (H3N2), A/New York/55/2004 (H3N2), A/Hiroshima/52/2005 (H3N2), A/Uruguay/716/2007 (H3N2), A/Victoria/210/2009 (H3N2), A/Victoria/361/2011 (H3N2), A/Texas/50/2012 (H3N2), A/New York/39/2012 (H3N2), A/Switzerland/9715293/2013 (H3N2), A/Vietnam/1194/2004 (H5N1), A/Indonesia/5/2005 (H5N1), A/Anhui/1/2005 (H5N1), A/Shanghai/2/2013 (H7N9), A/Anhui/1/2013 (H7N9), B/Shandong/7/97, B/Shanghai/361/2002, B/Malaysia/2506/2004, B/Florida/4/2006, B/Brisbane/60/2008, B/Wisconsin/1/2010, B/Massachusetts/2/2012, B/Phuket/3073/2013, and B/Texas/2/2013.

Other than influenza virus isolated from a living body as described above, influenza viruses to be used in the present invention may each be recombinant virus produced by adding modifications, such as attenuation, chicken egg growth adaptation, cell culture growth adaptation, modification into temperature-sensitive phenotype, and mucosal administration adaptation, so as to be applicable to influenza vaccine. In addition, as means for adding modifications, there are given, for example: a method involving introducing mutations into eight RNA segments, such as antigenic site and polymerase site, of influenza virus; a method involving producing an attenuated virus by cold-passage; and a method involving adding mutagen to the virus culture system.

In the present invention, the case in which two influenza viruses have similar antigenicities is expressed as having similar antigenicities. The expression "having similar antigenicities" generally refers to the fact that there is little genetic difference between the antigenic proteins of the viruses. The similarity in antigenicity may be generally examined by antigenicity analysis based on HI test or neutralization test. Specifically, when the homologous antibody titer of antiserum obtained from an animal infected with the first virus or an animal immunized with the virus differs from that against the second virus by 2 times or less, and the homologous antibody titer of antiserum obtained from an animal infected with the second virus or an animal immunized with the virus differs from that against the first virus by 2 times or less, it is determined that the first virus and the second virus have similar antigenicities. In antigenicity analysis of viruses of the H3N2 subtype in recent years, viruses different in antibody titer from each other by 4 times or less in a comparison by this method may be determined to have similar antigenicities.

The host to be used in the production method of the present invention may be an embryonated chicken egg, or may be cultured cells. When the embryonated chicken egg is used as the host, a specific pathogen-free (SPF) embryonated chicken egg may be used.

In the production method of the present invention, when the cultured cells are used as the host, the cultured cells may be any cultured cells that influenza virus can infect to be replicated. The cultured cells are preferably mammalian cells, and examples thereof include, but not limited to, hamster, bovine, primate (including human and monkey), and canine cells. More specific examples thereof include: MDCK cells derived from the Madin-Darby canine kidney; and Vero cells derived from the African green monkey kidney. The MDCK cells in the present invention are more specifically MDCK cells internationally deposited and identified by accession number NITE BP-02014. Such cells were domestically deposited to NITE Patent Microorganisms Depositary at the Biological Resource Center (2-5-8 Kazusakamatari, Kisarazu-shi, Chiba, Japan, postal code: 292-0818) with accession number NITE P-02014 on March 4, 2015, and then a request for conversion to an international deposit under the Budapest Treaty was made to NITE Patent Microorganisms Depositary at the Biological Resource Center.

The present invention also encompasses reassortant influenza virus produced by the production method for reassortant influenza virus of the present invention. Reassortant influenza virus produced by the production method of the present invention contains proteins derived from at least two kinds of influenza virus including an antigenic strain influenza virus and donor strain influenza virus. The antigenic strain influenza virus and donor strain influenza virus have similar antigenicities in some cases. Particularly when the antigenic strain influenza virus and donor strain influenza virus have similar antigenicities as described above, the stepwise reassortant virus production method of the present invention is applicable.

Reassortant influenza virus produced by the production method of the present invention may be used as a seed virus for influenza vaccine. For the step of purifying reassortant influenza virus, a known technique or any technique to be developed in the future may be used.

### Examples

To help understanding of the present invention, the present invention is specifically described below by way of Examples and Reference Examples, but the present invention is not limited to Examples and Reference Examples.

### (Example 1) Production of Influenza Virus (X) by Reassortment Method

In this Example, a production method for influenza virus (X) based on two reassortment steps using influenza viruses (1) to (3) is described. At first, reassortant influenza viruses were produced using influenza virus (2) as donor strain and using influenza virus (3) as an antigenic strain, and influenza virus (Y) was selected as reassortant donor strain. Next, influenza virus (X) was produced using reassortant donor strain and using influenza virus (1) as an antigenic strain. Influenza viruses are hereinafter described as follows: the strain (1), the strain (2), the strain (3), the strain (Y), and the strain (X).

### 1. Materials for Producing Strain (X)

### a) Viruses Used

Viruses used as the strains (1) to (3) and the strain (Y) are shown in Table 1. The strain (Y) is a strain selected from reassortant influenza viruses produced using the strain (2) as donor strain and the strain (3) as an antigenic strain.

**Table 1: Virus strains used**

| | Strain (1) | Strain (2) | Strain (3) | Strain (Y) |
|---|---|---|---|---|
| A | A/Yokohama/94/2015 (H1N1) pdm09 | A/Ibaraki/N12073/2011 (H1N1) pdm09 | A/Sapporo/38/2015 (H3N2) | A/Ibaraki/N12073/2011 (H1N1) pdm09 × A/Sapporo/38/2015 (H3N2) 6:2 Reassortant Virus |
| B | A/Sapporo/1/2016 (H1N1) pdm09 | A/Ibaraki/N12073/2011 (H1N1) pdm09 | A/Sapporo/38/2015 (H3N2) | A/Ibaraki/N12073/2011 (H1N1) pdm09 × A/Sapporo/38/2015 (H3N2) 6:2 Reassortant Virus |
| C | A/Wakayama-c/103/2015 (H3N2) | A/Ibaraki/N12232/2012 (H3N2) | A/California/7/2009 (H1N1) pdm09 | A/Ibaraki/N12232/2012 (H3N2) × A/California/7/2009 (H1N1) pdm09 6:2 Reassortant Virus |
| D | A/Iwate/37/2015 (H3N2) | A/Ibaraki/N12232/2012 (H3N2) | A/California/7/2009 (H1N1) pdm09 | A/Ibaraki/N12232/2012 (H3N2) × A/California/7/2009 (H1N1) pdm09 6:2 Reassortant Virus |

### b) Medium for virus culture

Eagle's MEM medium containing sodium hydrogen carbonate (20 mM) and 0.1×TrypLE Select was used.

MDCK cells internationally deposited and identified by accession number NITE BP-02014 were used as host cells. In this Example, these cells are hereinafter sometimes referred to simply as "MDCK cells".

A culture liquid of donor strain or influenza virus having antigenicity similar to that of donor strain was prepared at a concentration of from about 10⁷ to about 10⁸ TCID₅₀/mL, and 6 mL thereof was sprayed with a nebulizer to infect a ferret. On day 14 of the infection, blood was collected from the heart under anesthesia, and the collected blood was incubated at room temperature for 1 hour, at 36°C for 1 hour, and at 4°C for 24 hours. After that, the blood was centrifuged at about 700×g and room temperature for 10 minutes, and the supernatant was collected as infected ferret serum. The infected ferret serum was mixed with an equal amount of RDE(II) "SEIKEN" (Denka Seiken Co., Ltd.) to achieve a final dilution factor of 2 times. The mixture was incubated at 37°C for from 18 to 20 hours, and then incubated at 56°C for 1 hour to inactivate RDE. The resultant was used as anti-donor strain serum.

### 2. Production of Strain (X)

1) At first, the strain (Y) was produced by the first reassortment step method before the production of the strain (X) . The strain (2) was used as a donor strain for the production of the strain (Y), and the strain (3) was used as an antigenic strain therefor. With the use of the medium for virus culture, a 10⁷ TCID₅₀/mL donor strain (2) solution was prepared, and a 10⁷ TCID₅₀/mL antigenic strain (3) solution was prepared. Influenza virus concentration (infectious titer: TCID₅₀/mL) was confirmed in accordance with a method disclosed in Reference 1.
2) The antigenic strain (3) solution was dispensed into 3.5 cm dishes at 2 mL each. The dishes of 1) were placed in Spectrolinker XL-1000 (Spectronics Corporation, UV tubes: 254 nm, 8 W×5 tubes), the lids of the dishes were removed, and UV irradiation was performed at 500 J/m².
3) The MDCK cells were cultured in a 25 cm² flask to confluence (about 5×10⁶ cells/flask), the medium was removed, and the cells were inoculated with 200 µL of the antigenic strain subjected to the UV irradiation in 2), followed by culture at 34°C and 5% CO₂ for 30 minutes. After that, 10 mL of the medium for virus culture was added, and the cells were inoculated with 200 µL of donor strain (2) solution.
4) The cells were cultured at 34°C and 5% CO₂ for 2 days.
5) 100 µL of the resultant mixed culture liquid was mixed with 100 µL of the anti-strain (2) serum, and the mixture was incubated at 34°C for 1 hour.
6) MDCK cells were cultured in a fresh 25 cm² flask, the medium was changed to 10 mL of the medium for virus culture, and the cells were inoculated with the whole amount of 200 µL of the culture liquid treated with the anti-strain (2) serum in 5) above.
7) The cells were cultured at 34°C and 5% CO₂ for 2 days.
8) The resultant culture liquid was centrifuged (about 8,000×g, 5 minutes), and the supernatant was collected.
9) The centrifuged supernatant was diluted with the medium for virus culture to 10³ times, 10⁴ times, 10⁵ times, 10⁶ times, 10⁷ times, or 10⁸ times, and 6-well plates in which MDCK cells had been cultured to confluence were inoculated therewith at 100 µL/well. Two plates were inoculated.
10) The cells were cultured at 34°C and 5% CO₂ for 30 minutes.
11) 0.8% agarose-containing MEM medium (containing glutamine (4 mM) and 0.1×TrypLE Select) was overlaid at 3 mL/well. After drying in a safety cabinet, the cells were started to be cultured in an incubator.
12) The cells were cultured at 34°C and 5% CO₂ for 3 days.
13) 1.0% agarose-containing MEM medium (containing neutral red) was overlaid at 2 mL/well, followed by drying in a safety cabinet.
14) MDCK cells were cultured in a fresh 6-well plate, the medium was changed to 2 mL/well of the medium for virus culture, and plaques were isolated for each well to provide the strain (Y).
15) The cells were cultured at 34°C and 5% CO₂ for 3 days.
16) The culture liquid of the isolated plaque was centrifuged (about 8,000×g, 5 minutes), and the supernatant was stored at - 80°C. The resultant culture supernatant of the plaques was used as a strain (Y) culture liquid, and virus RNA was extracted and subjected to genetic analysis.
17) Next, the strain (X) serving as the target product of the present invention was produced by the second reassortment step method. Plaques were obtained by the same technique as in the production of the strain (Y) described above except that: the strain (Y) was used as donor strain; the strain (1) was used as the antigenic strain; and anti-donor strain serum was anti-strain (3) serum, and a culture supernatant of the plaques was produced as the strain (X).

### (Test Example 1) Genetic Analysis Results

Influenza viruses (X) produced in Example 1 by the two reassortment steps method were each subjected to genetic analysis. For each of influenza viruses (X) produced in four kinds of combination A to D shown in Table 1, three clones were subjected to the genetic analysis. For genetic analysis, RNA was extracted from the culture supernatant of each isolated plaque, and was reverse transcribed to synthesize cDNA, and all genome segments of the viruses were amplified by PCR in accordance with conventional methods, followed by simple purification. The resultant was used as a specimen and subjected to gene sequence analysis to determine which of the donor strain and the antigenic strain each genome segment was derived.

The genetic analysis results of the plaques are shown in Table 2 below. The results shown below confirmed that all influenza viruses (X) forming the plaques were reassortant influenza viruses. It was confirmed that all HA segments and many NA segments were derived from the strain (1), and at least one of the other genome segments was derived from the strain (Y).

**Table 2: Genetic analysis results of strain (X)**

| | Strain (1) | Strain (Y) | Plaque | PB2 | PB1 | PA | HA | NP | NA | M | NS |
|---|---|---|---|---|---|---|---|---|---|---|---|
| A | A/Yokohama/94/2015 (H1N1) pdm09 | A/Ibaraki/N12073/2011 (H1N1) pdm09 × A/Sapporo/38/2015 (H3N2) 6:2 Reassortant Virus | 1 | B | B | B | A | B | A | B | A |
| | | | 2 | B | B | B | A | A | A | A | B |
| | | | 3 | B | B | B | A | B | B | A | B |
| B | A/Sapporo/1/2016 (H1N1) pdm09 | A/Ibaraki/N12073/2011 (H1N1) pdm09 × A/Sapporo/38/2015 (H3N2) 6:2 Reassortant Virus | 1 | B | B | B | A | B | A | B | A |
| | | | 2 | A | B | B | A | A | A | B | A |
| | | | 3 | A | B | B | A | B | A | B | A |
| C | A/Wakayama-c/103/2015 (H3N2) | A/Ibaraki/N12232/2012 (H3N2) × A/California/7/2009 (H1N1) pdm09 6:2 Reassortant Virus | 1 | B | B | B | A | B | A | A | B |
| | | | 2 | B | B | B | A | B | A | B | B |
| | | | 3 | B | B | B | A | B | A | B | B |
| D | A/Iwate/37/2015 (H3N2) | A/Ibaraki/N12232/2012 (H3N2) × A/California/7/2009 (H1N1) pdm09 6:2 Reassortant Virus | 1 | B | B | B | A | B | A | B | B |
| | | | 2 | B | B | B | A | B | A | B | B |
| | | | 3 | B | B | B | A | B | A | B | B |

| | | | | | | | | | | | |
|---|---|---|---|---|---|---|---|---|---|---|---|
| A: Antigen, B: Backbone | | | | | | | | | | | |

### (Test Example 2) Infectious Titer Measurement

Some of obtained reassortant influenza viruses (X) and influenza virus (1) used as a parental strain were inoculated into MDCK cells that had been cultured in a 75 cm² flask to confluence. The virus inoculation was performed at moi of about 0.001, the cells were cultured at 34°C and 5% CO₂ for 3 days, and then the culture supernatant was collected and measured for the infectious titer.

The results of the infectious titer measurement are shown in Table 3 below. WT represents Wild Type. Some of produced reassortant influenza viruses (X) showed higher infectious titers than the parental strain virus (one using WT for Backbone in the table).

**Table 3: Infectious titer measurement result**

| | Antigen | Backbone | Titer (logTCID₅₀/ mL) |
|---|---|---|---|
| A | A/Yokohama/94/2015 (H1N1) pdm09 | WT | 7.76 |
| | | Ibaraki/N12073 bb Plaque #1 | 7.88 |
| | | Ibaraki/N12073 bb Plaque #2 | 8.11 |
| | | Ibaraki/N12073 bb Plaque #3 | 8.28 |
| B | A/Sapporo/1/2016 (H1N1) pdm09 | WT | 7.80 |
| | | Ibaraki/N12073 bb Plaque #1 | 7.90 |
| | | Ibaraki/N12073 bb Plaque #2 | 7.90 |
| C | A/Wakayama-c/103/2015 (H3N2) | WT | 6.60 |
| | | Ibaraki/N12232 bb Plaque #1 | 7.45 |
| | | Ibaraki/N12232 bb Plaque #2 | 7.28 |
| D | A/Iwate/37/2015 (H3N2) | WT | 7.21 |
| | | Ibaraki/N12232 bb Plaque #1 | 7.23 |

### Industrial Applicability

As described in detail above, according to the production method for reassortant influenza virus of the present invention, reassortant influenza virus that is a recombinant in which desired genome segments are arranged can be efficiently produced. According to the method of the present invention, influenza virus showing high growth potential can be produced early and efficiently, and hence a seed virus for influenza vaccine can be quickly produced.

## Claims

1. A production method for influenza virus (X) containing an antigenic protein (x), which is a production method for reassortant influenza virus, the production method comprising at least two reassortment steps including the following Step (A) and Step (B) making use of at least three kinds of influenza virus including following (1) to (3):
(1) the first influenza virus containing an antigenic protein (x);
(2) the second influenza virus having an antigenic protein (x') having similar antigenicity to that of influenza virus of (1); and
(3) the third influenza virus having an antigenic protein (y) having antigenicity different from that of influenza virus of (1) :
Step (A): the step including:
coculturing influenza virus (2) and influenza virus (3) by infecting a host therewith, to produce reassortant influenza viruses; and
selecting influenza virus (Y) having the antigenic protein (y) from reassortant influenza viruses; and
Step (B): the step including:
coculturing influenza virus (1) and influenza virus (Y) produced in Step (A) by infecting a host therewith, to produce reassortant influenza viruses; and
selecting influenza virus (X) having the antigenic protein (x) from reassortant influenza viruses.

2. The production method for influenza virus (X) according to claim 1, further comprising, before the coculturing influenza virus (2) and influenza virus (3) in Step (A), the step of treating influenza virus (3) so that influenza virus has initial infection ability and loses or is reduced in growth potential.

3. The production method for influenza virus (X) according to claim 1 or 2, further comprising, before the coculturing influenza virus (1) and influenza virus (Y) in Step (B), the step of treating influenza virus (1) so that influenza virus has initial infection ability and loses or is reduced in growth potential.

4. The production method for influenza virus (X) according to any one of claims 1 to 3, wherein the step of selecting influenza virus (Y) having the antigenic protein (y) in Step (A) comprises the step of bringing an antibody reactive to the antigenic protein (x') into contact therewith.

5. The production method for influenza virus (X) according to any one of claims 1 to 4, wherein the step of selecting influenza virus (X) having the antigenic protein (x) in Step (B) comprises the step of bringing an antibody reactive to the antigenic protein (y) into contact therewith.

6. The production method for influenza virus (X) according to any one of claims 1 to 5, wherein Step (A) includes selecting influenza virus (Y) having the antigenic protein (y) from reassortant influenza viruses.

7. The production method for influenza virus (X) according to any one of claims 1 to 6, wherein Step (B) includes selecting influenza virus (X) having the antigenic protein (x) from reassortant influenza viruses.

8. Influenza virus (X), which is produced by the production method of any one of claims 1 to 7.

9. Reassortant influenza virus, comprising proteins derived from at least two kinds of influenza virus including an antigenic strain influenza virus and donor strain influenza virus, wherein the antigenic strain influenza virus and donor strain influenza virus have similar antigenicities.
